(19) Europäisches Patentamt
European Patent Office
Office européen des brevets

(11) **EP 3 320 134 B1**

(12) **EUROPÄISCHE PATENTSCHRIFT**

(45) Veröffentlichungstag und Bekanntmachung des Hinweises auf die Patenterteilung:
**21.11.2018 Patentblatt 2018/47**

(51) Int Cl.:
***D04B 37/00*** *(2006.01)*    ***G01N 33/36*** *(2006.01)*
***G06T 11/00*** *(2006.01)*

(21) Anmeldenummer: **16775449.8**

(22) Anmeldetag: **09.09.2016**

(86) Internationale Anmeldenummer:
**PCT/CH2016/000115**

(87) Internationale Veröffentlichungsnummer:
**WO 2017/041191 (16.03.2017 Gazette 2017/11)**

(54) **VORHERSAGE DES AUSSEHENS EINER TEXTILEN FLÄCHE**

FORECASTING THE APPEARANCE OF A TEXTILE SURFACE

PRÉVISION DE L'APPARENCE D'UNE SURFACE TEXTILE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **10.09.2015 CH 13062015**

(43) Veröffentlichungstag der Anmeldung:
**16.05.2018 Patentblatt 2018/20**

(73) Patentinhaber: **Uster Technologies AG**
**8610 Uster (CH)**

(72) Erfinder: **STORZ, Rafael**
**8280 Kreuzlingen (CH)**

(74) Vertreter: **Pliska, Pavel**
**Uster Technologies AG**
**Sonnenbergstrasse 10**
**8610 Uster (CH)**

(56) Entgegenhaltungen:
**EP-A1- 0 568 700    EP-A1- 1 445 714**
**EP-A1- 1 452 985    WO-A1-2009/039668**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung liegt auf dem Gebiet der textilen Qualitätskontrolle. Sie betrifft ein Verfahren zur Vorhersage des Aussehens einer aus einem gegebenen Garn herzustellenden textilen Fläche, gemäss dem Oberbegriff des ersten Patentanspruchs. Sie betrifft auch eine Bilddatenbank von Bilddatensätzen zur Verwendung im erfindungsgemässen Verfahren, gemäss dem Oberbegriff eines weiteren unabhängigen Patentanspruchs. Ferner betrifft die Erfindung ein Verfahren zur Erzeugung der erfindungsgemässen Bilddatenbank, gemäss dem Oberbegriff eines weiteren unabhängigen Patentanspruchs.

STAND DER TECHNIK

Schon immer bestand in der Textilindustrie der Bedarf nach einer schnellen Vorhersage des Aussehens einer textilen Fläche, die aus einem gegebenen Garn hergestellt wird. Das Weben oder Stricken einer realen textilen Fläche aus dem Garn ist zu aufwändig und dauert zu lange. Einen brauchbaren Eindruck kann eine Garnschautafel vermitteln, die durch dichtes Aufwickeln des Garns auf eine rechteckige oder trapezförmige Tafel entsteht.

Seit elektronische Garnprüfgeräte und Computer zur Verfügung stehen, wurde versucht, mit einem Garnprüfgerät mindestens einen Parameterwert des Garns zu messen, die Messresultate in den Computer einzugeben, daraus das Aussehen einer textilen Fläche zu berechnen und das berechnete Aussehen als Simulation auf einem Ausgabegerät darzustellen. Solche Simulationsverfahren sind z. B. in den Schriften US-5,671,061 A, WO-97/31262 A1, WO-98/16823 A1, US-6,928,335 B1 und EP-1'006'225 A2 beschrieben. Sie haben zwei Nachteile. Der erste Nachteil ist operativer Art: Das Programmieren ist zeitaufwändig, das Berechnen der Simulation ist rechenaufwändig, und sowohl bei der Berechnung als auch bei der Darstellung der Simulation können sich Artefakte einschleichen. Der zweite Nachteil ist grundsätzlicher Art: Jede Simulation beruht auf einem ausgemessenen Garnabschnitt endlicher Länge, d. h. auf einer bestimmten Stichprobe, die nicht ideal zu sein braucht. Je nach Stichprobe können die Simulationen unterschiedlich ausfallen, selbst wenn sie auf Stichproben desselben Garntyps beruhen.

Die US-6,510,734 B1 schlägt vor, zwei Simulationen zum Vergleich auszugeben: eine, die vom gegebenen realen Garn ausgeht, und eine, die von einem Bildgarn ausgeht. Die für die Simulation benötigten Parameter des Bildgarns werden durch Messung eines wirklich existierenden Bildgarns oder durch Berechnung aus gegebenen statistischen Werten gewonnen. Im letzteren Fall könnte zumindest der zweite oben erwähnte Nachteil beseitigt werden, der erste hingegen nicht.

Gemäss der EP-1'452'985 A1 wird ein Bild eines gestreckten Garns aufgenommen und in Abschnitte unterteilt. Die Bildabschnitte werden dann mittels Bildverarbeitung in die Form einer Strickmasche umgeformt. Aus der so erhaltenen Strickmasche wird ein Bild eines Gestricks erzeugt und ausgegeben.

Die EP-0'568'700 A1 befasst sich mit einem System für den Entwurf von Gestrick und mit einem Verfahren zur Erzeugung von Strickdaten dafür. Sie erwähnt das Abspeichern von Bildinformation, die eine Maschenstruktur angibt.

Die EP-1'445'714 A1 beschreibt ein Verfahren und einer Vorrichtung zum Stricken. Sie lehrt, mehrere Bilder von existierenden Gestricken aufzunehmen und sie mittels Bildverarbeitung zusammenzusetzen. Das synthetische, zusammengesetzte Bild wird auf einem Bildschirm ausgegeben.

Die WO-2009/039668 A1 befasst sich mit einem System und einem Verfahren für den Entwurf von gestrickten Objekten. In einem bestimmten Verfahrensschritt werden auf einem Bildschirm Bilder von aus verschiedenen Garnen hergestellten Gestricken ausgegeben, von denen der Benutzer eines auswählen kann.

Ein Verfahren und eine Vorrichtung zur Prüfung von Garn im Textillabor sind z. B. aus der US-2008/0209998 A1 bekannt. Diese Vorrichtung arbeitet nach dem Durchlaufprinzip, d. h. das zu prüfende Garn wird in einem einzigen Prüfdurchlauf von einer Garnspule abgezogen und durchläuft in serieller Weise in der Vorrichtung eine Anordnung von Sensoren, um die zu bestimmenden Parameter zu messen. Die Auswertung der Sensorsignale erfolgt in der Regel in einer mit der Vorrichtung verbundenen Steuer- und Auswerteeinheit, die als Arbeitsplatzrechner ausgestaltet sein kann.

Auch mit einem in der Garnproduktion eingesetzten Garnreiniger, wie er z. B. in der WO-2012/051730 A1 beschrieben ist, lässt sich mindestens ein Parameterwert von Garn bestimmen.

DARSTELLUNG DER ERFINDUNG

Es ist eine Aufgabe der vorliegenden Erfindung, ein Verfahren zur Vorhersage des Aussehens einer textilen Fläche anzugeben, das die obigen Nachteile vermeidet. Insbesondere soll das Verfahren gute, wirklichkeitsnahe Resultate ohne Artefakte liefern. Es soll möglichst einfach, schnell ausführbar und dynamisch erweiterbar sein. Weitere Aufgaben der Erfindung bestehen darin, eine Bilddatenbank zur Verwendung im erfindungsgemässen Verfahren und ein Verfahren zur Erzeugung der Bilddatenbank zu schaffen.

Diese und andere Aufgaben werden durch die erfindungsgemässen Verfahren, wie sie in den unabhängigen Patentansprüchen definiert sind, gelöst. Vorteilhafte Ausführungsformen sind in den abhängigen Patentansprüchen angegeben.

Die Erfindung basiert auf der Idee, vorgängig eine textile Fläche aus einem Bildgarn herzustellen. Eine Bilddatenbank von Bilddatensätzen wird erzeugt, wobei jeder Bilddatensatz ein Bild der textilen Fläche und einen Bildvektor aus Metadaten, die dem Bild zugeordnet sind, beinhaltet. Aus Eingangsdaten, die sich auf das gegebene Garn beziehen, wird ein Gamvektor gebildet. Aufgrund von Abständen zwischen dem Garnvektor und den Bildvektoren wird ein passendes Bild aus der Datenbank ausgewählt und ausgegeben. Dasjenige Bild wird zur Ausgabe ausgewählt, dessen Metadaten den Eingangsdaten am nächsten kommen. Für welche Metadaten dies zutrifft, wird anhand einer geeignet definierten Metrik bestimmt.

Unter dem Begriff "textile Fläche" werden hier sämtliche flächigen Gebilde zusammengefasst, die aus mindestens einem Garn herstellbar sind. Beispiele für textile Flächen sind Gewebe, Gestricke, Gewirke, aber auch Gamschautafeln.

Das erfindungsgemässe Verfahren dient zur Vorhersage des Aussehens einer aus einem gegebenen Garn herzustellenden textilen Fläche. Eine Bilddatenbank von Bilddatensätzen wird bereitgestellt, in welcher Bilddatenbank jeder Bilddatensatz ein Bild einer aus mindestens einem Bildgarn hergestellten textilen Fläche und eine dem Bild zugeordnete Metadatengruppe beinhaltet. Jede der Metadatengruppen beinhaltet eine erste Menge von Daten, die einen Bildvektor in einem Vektorraum bilden. Auf dem Vektorraum wird eine Metrik definiert, die je zwei Vektoren des Vektorraums einen Abstand zuordnet. Eine sich zumindest teilweise auf das gegebene Garn beziehende Eingangsdatengruppe wird gebildet, die eine zweite Menge von Daten beinhaltet, welche Daten einen Gamvektor in demselben Vektorraum bilden. Abstände zwischen dem Garnvektor einerseits und den Bildvektoren andererseits werden bestimmt und miteinander verglichen. Aufgrund des Vergleichs wird ein Bilddatensatz, und vorzugsweise genau ein Bilddatensatz, aus der Bilddatenbank ausgewählt, und das in dem ausgewählten Bilddatensatz enthaltene Bild wird ausgegeben.

Der Ausdruck "Datengruppe" bezeichnet in der vorliegenden Schrift nicht notwendigerweise eine Mehrzahl von Daten. Eine Datengruppe kann, muss aber nicht, aus einem einzigen Zeichen oder Symbol bestehen.

Der Fachmann kennt den Begriff des Vektorraums, der in der Technik eine grosse Bedeutung hat, bspw. aus dem Lehrbuch "Höhere Mathematik für Ingenieure", Band II Lineare Algebra, 2. Auflage, Springer Fachmedien, 1990. Insbesondere kennt der Fachmann den n-dimensionalen Vektorraum $R^n$ über der Menge R der reellen Zahlen, dessen Dimension n eine natürliche Zahl grösser oder gleich eins ist. Die natürliche Zahl n ist die Mächtigkeit der ersten und der zweiten Menge von Daten. Der Bildvektor und der Garnvektor sind von derselben Dimension n und bestehen aus einander jeweils entsprechenden Komponenten, die sich auf dieselbe Eigenschaft beziehen. So kann sich z. B. eine erste Komponente des Bildvektors auf einen Variationskoeffizienten der Garnmasse des Bildgarns und eine erste Komponente des Garnvektors auf einen Variationskoeffizienten der Garnmasse des gegebenen Garns beziehen, usw.

Die Eingangsdatengruppe und/oder die Metadatengruppen können Daten beinhalten, die sich auf mindestens einen der folgenden Aspekte beziehen: mindestens ein Parameterwert eines Garnparameters des gegebenen Garns bzw. des Bildgarns, für die Herstellung des gegebenen Garns bzw. des Bildgarns verwendetes Rohmaterial, Herstellung des gegebenen Garns bzw. des Bildgarns, Herstellung der textilen Fläche, Erzeugung des Bildes.

In einer Ausfiihrungsform des Verfahrens beinhalten die Eingangsdatengruppe und jede der Metadatengruppen jeweils einen Parameterwert mindestens eines Garnparameters des gegebenen Garns bzw. des Bildgarns. Der mindestens eine Garnparameter bezieht sich z. B. auf mindestens eine der folgenden Eigenschaften des gegebenen Garns bzw. des Bildgarns: Masse, Querdimension, Anzahl Nissen, Garnnummer, Haarigkeit, Fremdstoffgehalt, Reissfestigkeit, Dehnung, Drehung, Reibung. Der mindestens eine Garnparameter braucht nicht direkt das gegebene Garn zu charakterisieren, sondern kann alternativ oder zusätzlich ein Rohmaterial oder ein Zwischenprodukt, das für die Herstellung des gegebenen Garns verwendet wurde, charakterisieren. Auch in diesem Fall bezieht es sich ja auf das gegebene Garn. Eine detaillierte Zusammenstellung von über 300 Garnparametern, die in dieser Ausfuhrungsform des erfindungsgemässen Verfahrens verwendet werden können, gibt der Anwendungsbericht "USTER® *LABORATORY SYSTEMS. Description of all quality parameters measured by Uster Technologies fiber and yarn testing equipment*", SE-562, Uster Technologies AG, Version 4, 2014.

Eine Messung am gegebenen Garn kann durchgeführt werden, und der mindestens eine Parameterwert des gegebenen Garns kann aus mindestens einem Resultat der Messung und vorzugsweise durch eine anschliessende statistische Auswertung von Resultaten der Messung bestimmt werden.

Die Eingangsdatengruppe kann Daten beinhalten, die sich auf mindestens einen der folgenden Aspekte beziehen: für die Herstellung des gegebenen Garns verwendetes Rohmaterial, Herstellung des gegebenen Garns, vorgesehene Herstellung der textilen Fläche, Erzeugung des Bildes. Die Definition der Metrik kann dann von den genannten Daten abhängig sein.

Es wird z. B. derjenige Bilddatensatz ausgewählt, für dessen Bildvektor der Abstand am kleinsten ist.

Zusätzlich zu dem Bild kann ein aus dem Abstand berechneter Gütewert ausgegeben werden, der ein Mass für eine Güte der Vorhersage ist.

In einer Ausführungsform enthält die Bilddatenbank eine Menge von Bilddatensätzen, die Bilder verschiedener textiler Flächen, aber Metadatengruppen mit gleichen ersten Mengen von Daten beinhalten. Die textilen Flächen mit gleichen ersten Mengen von Daten können sich durch mindestens einen der folgenden Unterscheidungsaspekte voneinander unterscheiden: Herstellungsverfahren der textilen Fläche, Typ einer die textile Fläche herstellenden Maschine. Der mindestens eine Unterscheidungsaspekt ist vorzugsweise in den entsprechenden Metadatengruppen abgebildet.

In einer Ausführungsform enthält die Bilddatenbank eine Menge von Bilddatensätzen, die verschiedene Bilder derselben textilen Fläche beinhalten. Die verschiedenen Bilder derselben textilen Fläche können jeweils durch ein fotografisches Verfahren aufgenommen worden sein. Sie können sich durch mindestens einen der folgenden Unterscheidungsaspekte voneinander unterscheiden: Art der Beleuchtung der textilen Fläche bei der Bildaufnahme, gegenseitige Lage einer Kamera und der textilen Fläche bei der Bildaufnahme, bei der Bildaufnahme eingestellte fototechnische Parameter. Der mindestens eine Unterscheidungsaspekt ist vorzugsweise in den entsprechenden Metadatengruppen abgebildet.

Es ist vorteilhaft, wenn die Bilddatenbank mindestens 100, vorzugsweise mindestens 1000 und bspw. mindestens 10'000 Bilddatensätze enthält.

Die erfindungsgemässen Verfahrensschritte der Vergleiche der Bestimmung und des Vergleichs der Abstände, der Auswahl eines Bilddatensatzes und der Ausgabe des Bildes werden vorzugsweise von einem Computer ausgeführt. Dabei kann Cloud Computing angewendet werden, indem die Eingangsdatengruppe von einem lokalen Computer über ein Netzwerk an mindestens einen entfernten Computer übermittelt wird, der Vergleich und die Auswahl durch den mindestens einen entfernten Computer erfolgt, das ausgewählte Bild über das Netzwerk von dem mindestens einen entfernten Computer an den lokalen Computer übermittelt wird und das ausgewählte Bild von dem lokalen Computer ausgegeben wird.

Die Erfindung umfasst auch ein Computerprogrammprodukt mit auf einem maschinenlesbaren Träger gespeichertem Programmcode zur Durchführung im vorangehenden Abschnitt genannten Verfahrensschritte, wenn das Computerprogrammprodukt auf einem Computer abläuft.

Die Erfindung bezieht sich auch auf eine Bilddatenbank von Bilddatensätzen zur Verwendung im erfindungsgemässen Verfahren. Jeder Bilddatensatz beinhaltet ein Bild einer aus mindestens einem Bildgarn hergestellten textilen Fläche und eine dem Bild zugeordnete Metadatengruppe. Jede der Metadatengruppen beinhaltet eine erste Menge von Daten, die einen Bildvektor in einem Vektorraum bilden, wobei auf dem Vektorraum eine Metrik definierbar ist, die je zwei Vektoren des Vektorraums einen Abstand zuordnet. Die Bilddatenbank enthält mindestens 100, vorzugsweise mindestens 1000 und bspw. mindestens 10'000 Bilddatensätze.

Die Metadatengruppen können Daten beinhalten, die sich auf mindestens einen der folgenden Aspekte beziehen: mindestens ein Parameterwert eines Garnparameters des Bildgarns, für die Herstellung des Bildgarns verwendetes Rohmaterial, Herstellung des Bildgarns, Herstellung der textilen Fläche, Erzeugung des Bildes.

In einer Ausführungsform beinhaltet jede der Metadatengruppen jeweils einen Parameterwert mindestens eines Garnparameters des Bildgarns. Der mindestens eine Garnparameter bezieht sich z. B. auf mindestens eine der folgenden Eigenschaften des Bildgarns: Masse, Querdimension, Anzahl Nissen, Garnnummer, Haarigkeit, Fremdstoffgehalt, Reissfestigkeit, Dehnung, Drehung, Reibung.

In einer Ausführungsform enthält die Bilddatenbank eine Menge von Bilddatensätzen, die Bilder verschiedener textiler Flächen, aber Metadatengruppen mit gleichen ersten Mengen von Daten beinhalten. Die textilen Flächen mit gleichen ersten Mengen von Daten können sich durch mindestens einen der folgenden Unterscheidungsaspekte voneinander unterscheiden: Herstellungsverfahren der textilen Fläche, Typ einer die textile Fläche herstellenden Maschine, und wobei der mindestens eine Unterscheidungsaspekt in den entsprechenden Metadatengruppen abgebildet ist.

In einer Ausführungsform enthält die Bilddatenbank eine Menge von Bilddatensätzen, die verschiedene Bilder derselben textilen Fläche beinhalten. Die verschiedenen Bilder wurden jeweils durch ein fotografisches Verfahren aufgenommen. Sie können sich durch mindestens einen der folgenden Unterscheidungsaspekte voneinander unterscheiden: Art der Beleuchtung der textilen Fläche bei der Bildaufnahme, gegenseitige Lage einer Kamera und der textilen Fläche bei der Bildaufnahme, bei der Bildaufnahme eingestellte fototechnische Parameter, und wobei der mindestens eine Unterscheidungsaspekt in den entsprechenden Metadatengruppen abgebildet ist.

Ferner bezieht sich die Erfindung auch auf ein Verfahren zur Erzeugung einer Bilddatenbank von Bilddatensätzen zur Verwendung im erfindungsgemässen Verfahren. Bilder mindestens einer aus mindestens einem Bildgarn hergestellten textilen Fläche werden erzeugt. Bilddatensätze werden gebildet, wobei jeder Bilddatensatz eines der Bilder und eine dem Bild zugeordnete Metadatengruppe beinhaltet. Jede der Metadatengruppen beinhaltet eine erste Menge von Daten, die einen Bildvektor in einem Vektorraum bilden, wobei auf dem Vektorraum eine Metrik definierbar ist, die je zwei Vektoren des Vektorraums einen Abstand zuordnet. Die Bilddatenbank wird aus mindestens 100, vorzugsweise mindestens 1000 und bspw. mindestens 10'000 Bilddatensätzen erzeugt.

In einer Ausfuhrungsform beinhalten die Metadatengruppen Daten, die sich auf mindestens einen der folgenden Aspekte beziehen: mindestens ein Parameterwert eines Garnparameters des Bildgarns, für die Herstellung des Bildgarns verwendetes Rohmaterial, Herstellung des Bildgarns, Herstellung der textilen Fläche, Erzeugung der Bilder.

In einer Ausführungsform beinhaltet jede der Metadatengruppen jeweils einen Parameterwert mindestens eines Garnparameters des Bildgarns. Der mindestens eine Garnparameter bezieht sich auf mindestens eine der folgenden Eigenschaften des Bildgarns: Masse, Querdimension, Anzahl Nissen, Garnnummer, Haarigkeit, Fremdstoffgehalt, Reissfestigkeit, Dehnung, Drehung, Reibung. Zur Bestimmung des Parameterwertes kann eine Messung am Bildgarn durchgeführt werden. Der mindestens eine Parameterwert des Bildgarns wird aus mindestens einem Resultat der Messung und vorzugsweise durch eine anschliessende statistische Auswertung von Resultaten der Messung bestimmt.

In einer Ausführungsform werden mehrere verschiedene textile Flächen aus demselben Bildgarn hergestellt, so dass die Bilddatenbank eine Menge von Bilddatensätzen enthält, die Bilder verschiedener textiler Flächen, aber Metadatengruppen mit gleichen ersten Mengen von Daten beinhalten. Die textilen Flächen mit gleichen ersten Mengen von Daten können sich durch mindestens einen der folgenden Unterscheidungsaspekte voneinander unterscheiden: Herstellungsverfahren der textilen Fläche, Typ einer die textile Fläche herstellenden Maschine, und wobei der mindestens eine Unterscheidungsaspekt in den entsprechenden Metadatengruppen abgebildet ist.

In einer Ausführungsform werden mehrere verschiedene Bilder derselben textilen Fläche erzeugt.

Die Bilder werden vorzugsweise durch ein fotografisches Verfahren aufgenommen. Alternativ können die Bilder z. B. mittels einer Computersimulation erzeugt werden.

Wenn die verschiedenen Bilder derselben textilen Fläche jeweils durch ein fotografisches Verfahren aufgenommen werden, können sie sich durch mindestens einen der folgenden Unterscheidungsaspekte voneinander unterscheiden: Art der Beleuchtung der textilen Fläche bei der Bildaufnahme, gegenseitige Lage einer Kamera und der textilen Fläche bei der Bildaufnahme, bei der Bildaufnahme eingestellte fototechnische Parameter, und wobei der mindestens eine Unterscheidungsaspekt in den entsprechenden Metadatengruppen abgebildet ist.

Da erfindungsgemäss Bilder von echten textilen Flächen ausgegeben werden, sind die Resultate des erfindungsgemässen Verfahrens wirklichkeitsnah. Keine noch so gute rechnerische Simulation kann derart realistische Resultate wie das erfindungsgemässe Verfahren liefern. Ausserdem ist das erfindungsgemässe Verfahren einfach und schnell ausführbar, weil es hauptsächlich Daten miteinander vergleicht, was für einen Computer eine relativ einfache Aufgabe ist.

Die erfindungsgemässe Bilddatenbank ist dynamisch veränderbar. Sie kann bei Bedarf jederzeit mit Bilddatensätzen erweitert oder ergänzt werden, die sich auf weitere Parameter, Garne, textile Flächen, Herstellungsverfahren, Aufnahmebedingungen etc. beziehen. Nicht mehr aktuelle Bilddatensätze können aus der Bilddatenbank gelöscht werden. Änderungen der Bilddatenbank können einerseits durch einen Bilddatenbankanbieter erfolgen. Andererseits kann sogar ein Bilddatenbankanwender seine Bilddatenbank verändern. Er kann z. B. eigene Bilddatensätze hinzufügen, solange diese gewissen Anforderungen, bspw. einem bestimmten Datenformat, genügen.

AUFZÄHLUNG DER ZEICHNUNGEN

Nachfolgend wird die Erfindung anhand der Zeichnungen detailliert erläutert.

Figur 1 zeigt schematisch eine Ausführungsform einer Vorrichtung zur Ausführung des erfindungsgemässen Verfahrens.

Figur 2 zeigt schematisch einen Vergleich einer Eingangsdatengruppe mit einer Metadatengruppe einer erfindungsgemässen Datenbank.

Figuren 3-5 zeigen Beispiele für Bilder von textilen Flächen zur Verwendung im erfindungsgemässen Verfahren.

Figur 6 zeigt ein Beispiel eines Vektorraums, in dem Parameterwerte eines gegebenen Garns mit Parameterwerten von Bildgarnen verglichen werden können.

Figur 7 zeigt ein Flussdiagramm einer Ausführungsform des erfindungsgemässen Verfahrens zur Erzeugung einer Bilddatenbank.

Figur 8 zeigt ein Flussdiagramm einer ersten Ausführungsform des erfindungsgemässen Verfahrens zur Vorhersage des Aussehens einer textilen Fläche.

Figur 9 zeigt ein Flussdiagramm einer zweiten Ausfährungsform des erfindungsgemässen Verfahrens zur Vorhersage des Aussehens einer textilen Fläche.

AUSFÜHRUNG DER ERFINDUNG

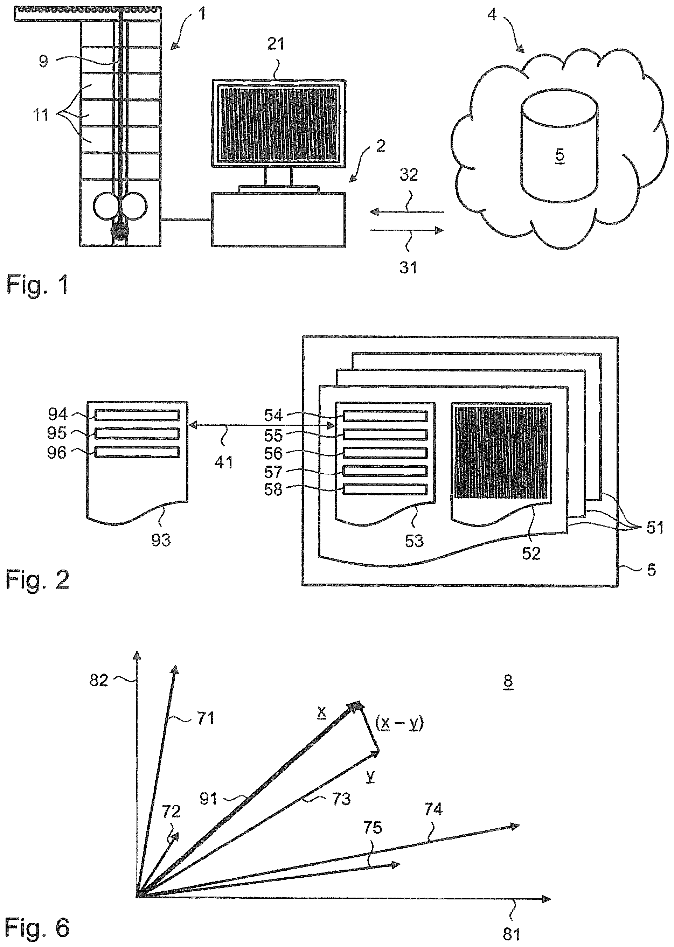

**Figur 1** zeigt eine Ausführungsform einer Vorrichtung zur Ausführung des erfindungsgemässen Verfahrens. Sie beinhaltet ein im Textillabor eingesetztes Garnprüfgerät 1, wie es aus der US-2008/0209998 A1 bekannt ist, zum Ausmessen eines gegebenen Garns 9. Das Garnprüfgerät 1 kann verschiedene Module 11 zum Messen verschiedener Eigenschaften des Garns 9 beinhalten. Alternativ kann das Ausmessen des gegebenen Garns 9 mittels eines in der Garnproduktion eingesetzten Garnreinigers, wie er aus der WO-2012/051730 A1 bekannt ist, oder mittels einer anderen Messvorrichtung erfolgen. In einer anderen Ausführungsform kann alternativ oder zusätzlich zur direkten Ausmessung des Garns 9 ein Rohmaterial oder ein Zwischenprodukt, das für die Herstellung des Garns 9 verwendet wurde, mit einem entsprechenden Prüfgerät ausgemessen werden, bspw. Rohbaumwollfasern mit einem Faserprüfgerät.

Resultate der Messungen werden an einen lokalen Computer 2, z. B. einen Arbeitsplatzrechner, übermittelt, der daraus jeweils einen Parameterwert 94, 95 (siehe Figur 2) mindestens eines Garnparameters bestimmt. Die Bestimmung des mindestens einen Parameterwertes 94, 95 erfolgt vorzugsweise durch eine statistische Auswertung der Messresultate. Der mindestens eine Garnparameter kann sich z. B. auf mindestens eine der folgenden Eigenschaften des gegebenen Garns 9 beziehen: Masse, Querdimension, Anzahl Nissen, Garnnummer, Haarigkeit, Fremdstoffgehalt, Reissfestigkeit, Dehnung, Drehung, Reibung.

Die in Figur 1 dargestellte Ausführungsform wendet Cloud Computing an. Der mindestens eine Parameterwert 94, 95 des Garns 9 wird von dem lokalen Computer 2 über ein Netzwerk an mindestens einen entfernten Computer 4 übermittelt, was mit einem Pfeil 31 dargestellt ist. Der mindestens eine entfernte Computer ist in Figur 1 als metaphorische "Wolke" 4, die für eine Infrastruktur der Informationstechnologie steht, eingezeichnet.

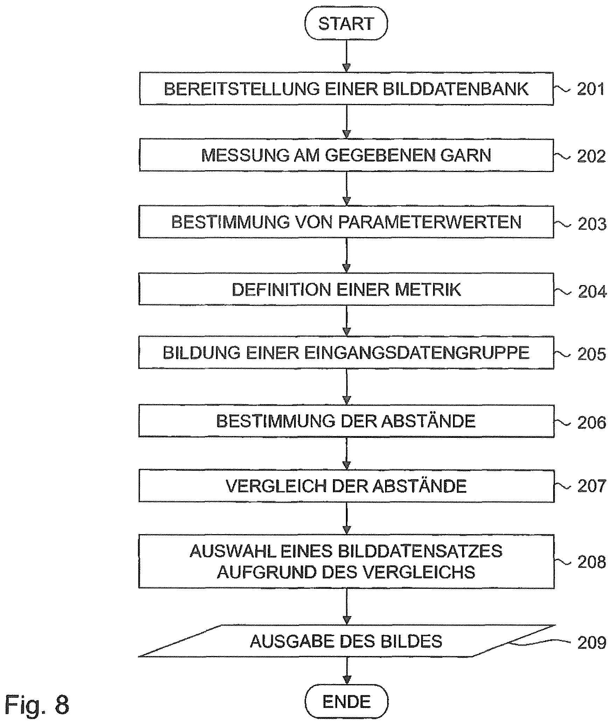

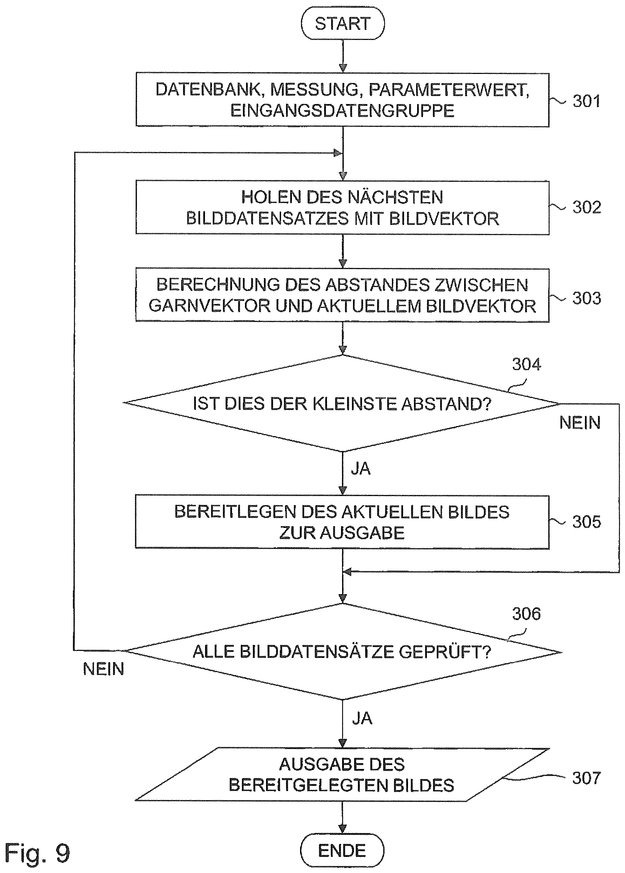

Auf Ausführungsformen des Verfahrens zur Vorhersage des Aussehens einer aus dem gegebenen Garn 9 herzustellenden textilen Fläche wird unten anhand der Figuren 8 und 9 näher eingegangen.

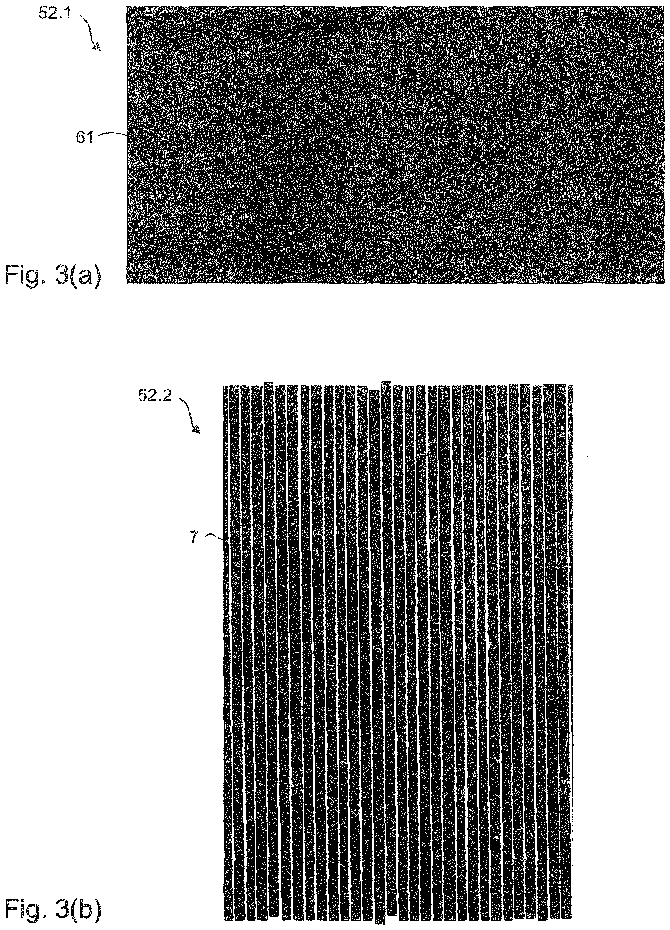

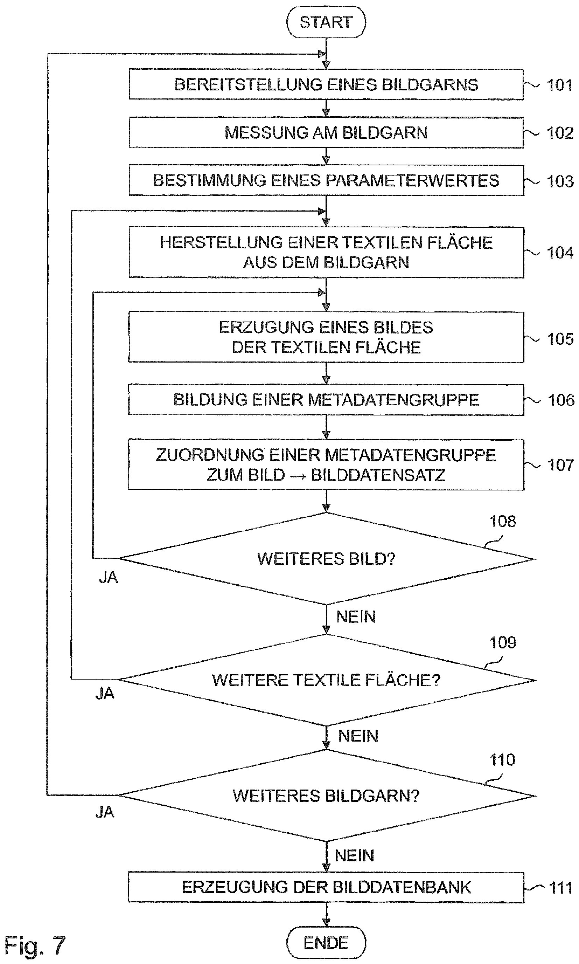

Die Wolke 4 kann unter anderem eine auch in der **Figur 2** dargestellte Bilddatenbank 5 von Bilddatensätzen 51 beinhalten. Jeder Bilddatensatz 51 beinhaltet ein Bild 52 einer aus mindestens einem Bildgarn 7 (siehe Figur 3(b)) hergestellten textilen Fläche 61-64 (siehe Figuren 3-5) und eine dem Bild 52 zugeordnete Metadatengruppe 53. Die Bilddatenbank 5 wird vorgängig erzeugt. Eine Ausführungsform des Verfahrens zur Erzeugung der Bilddatenbank 5 wird unten anhand von Figur 7 beschrieben. Die Metadatengruppen 53 beinhalten Daten, die sich auf mindestens einen der folgenden Aspekte beziehen: mindestens ein Parameterwert 54, 55 eines Garnparameters des Bildgarns 7, für die Herstellung des Bildgarns 7 verwendetes Rohmaterial, Herstellung des Bildgarns 7, Herstellung der textilen Fläche 61-64, Erzeugung des Bildes 52. In dem einfachen Beispiel von Figur 2 sind schematisch die folgenden Daten 54-58 eingezeichnet, die jede Metadatengruppe 53 enthalten kann:

- Zwei Parameterwerte 54, 55, die sich auf zwei verschiedene Garnparameter des Bildgarns 7 beziehen, bspw. auf den Variationskoeffizienten $CV_m$ der Garnmasse und die Haarigkeit H. Die Garnparameter stimmen mit denjenigen überein, die für die Charakterisierung des gegebenen Garns 9 verwendet werden.
- Spinnverfahren 56 des Bildgarns 7: bspw. Ringspinnen.
- Herstellung 57 der textilen Fläche 62: bspw. Weben.
- Erzeugung 58 des Bildes 52: bspw. Durchlicht.

Die Daten 54-58 sind in einem geeigneten digitalen Datenformat gespeichert, worauf hier nicht detailliert eingegangen zu werden braucht. Dasselbe gilt für die Daten des Bildes 52. In einer Ausführungsform enthält die Bilddatenbank 5 mindestens 100, vorzugsweise mindestens 1000 und bspw. mindestens 10'000 Bilddatensätze 51. Diese Zahlen basieren auf den folgenden Überlegungen. Ein Bilddatensatz 51 kann durch Daten aus den drei folgenden Ebenen charakterisiert werden:

- Garnkategorie. Diese ist durch das Material und das Herstellungsverfahren des Bildgarns 7 sowie durch das Herstellungsverfahren der textilen Fläche 61-64 gegeben. Beispiel: 100 % Baumwolle, Ringgarn, kardiert, auf Kreuzspule, zum Stricken.
- Abstufung der Parameter, d. h. Anzahl der diskreten Parameterwerte, innerhalb einer Kategorie. Beispiel: zwei Parameter, von denen je 10 diskrete Werte gegeben sind, ergeben 20 Parameterwerte in einer Kategorie.
- Bildart. Diese ist hauptsächlich durch das Herstellungsverfahren der textilen Fläche 61-64 und durch das Aufnahmeverfahren des Bildes 52 gegeben. Beispiel: Gewebe im Auflicht.

Die folgende Tabelle zeigt in den drei Spalten Beispiele für kleine, mittlere und grosse Anzahlen Elemente in jeder der obigen Ebenen. Die in der letzten Zeile angegebenen Werte für die Gesamtzahl der benötigten Bilddatensätze 51 ergeben sich jeweils aus der Multiplikation der Werte aus den drei vorangehenden Zeilen.

|  | Klein | Mittel | Gross |
|---|---|---|---|
| Garnkategorien | 10 | 25 | 50 |
| Abstufungen | 10 | 20 | 50 |
| Bildarten | 1 | 2 | 4 |
| **Gesamtzahl** | **100** | **1000** | **10'000** |

Die Parameterwerte 94, 95 des gegebenen Garns 9 sind in einer Gruppe 93 von Daten, die sich zumindest teilweise auf das gegebene Garn 9 beziehen, zusammengefasst. Diese Gruppe 93 wird in der vorliegenden Schrift "Eingangsdatengruppe" genannt, weil es sich um Eingangsdaten des erfindungsgemässen Verfahrens handelt. Nebst den Parameterwerten 94, 95 enthält die Eingangsdatengruppe 93 weitere Daten 96 wie z. B. die vorgesehene Verwendung des Garns 9.

In der Wolke 4 werden die Daten 94, 95 des gegebenen Garns 9 mit den Daten 54, 55 der Bildgarne 7, auf die sich die Bilder 52 beziehen, verglichen, was in Figur 2 mit einem Doppelpfeil 41 angedeutet ist. Ein Beispiel für den Vergleich wird unten anhand der Figur 6 erläutert. Der Vergleich 41 erfolgt, indem in einem entsprechenden Vektorraum Abstände zwischen zwei Vektoren bestimmt und miteinander verglichen werden, nämlich einem ersten Vektor, des aus den Daten 94, 95 des gegebenen Garns 9 gebildet ist, und zweiten Vektoren, die aus Daten 54, 55 aus den Metadatensätzen 53 gebildet sind.

Aufgrund des Vergleichs 41 wird ein bestimmter Bilddatensatz 51 aus der Bilddatenbank 5 ausgewählt. Das Bild 52 aus dem ausgewählten Bilddatensatz 51 wird über das Netzwerk an den lokalen Computer 2 übermittelt, was in Figur 1 mit einem Pfeil 32 dargestellt ist. Das übermittelte Bild 52 wird auf einer mit dem lokalen Computer 2 verbundenen Ausgabeeinheit 21, z. B. einem Bildschirm oder einem Drucker, ausgegeben.

Einer der Vorteile des Cloud Computing besteht darin, dass die grosse Datenmenge in der Datenbank 5 in der Wolke 4 gespeichert und die meisten Verfahrensschritte in der Wolke 4 durchgeführt werden. Dadurch wird der lokale Computer 2 entlastet. Dank den geringen technischen Anforderungen an den lokalen Computer 2 kann dieser auch als tragbares elektronisches Gerät, z. B. als Smartphone, ausgeführt sein. Ein weiterer Vorteil des Cloud Computing ist, dass die Datenbank 5 zentral gepflegt und erweitert werden kann. Dennoch kann das erfindungsgemässe Verfahren anstelle des Cloud Computing vollständig lokal, z. B. auf dem lokalen Computer 2, durchgeführt werden.

Die Figuren 3-5 zeigen Beispiele für Bilder 52.1-52.6 von textilen Flächen 61-64, wie sie im erfindungsgemässen Verfahren verwendet werden können.

In den Figuren 3 sind Bilder 52.1, 52.2 ein und derselben Garnschautafel 61, die eine textile Fläche ist, dargestellt. Der Unterschied zwischen den beiden Bildern 52.1, 52.2 besteht in der Aufnahmedistanz. Das Bild 52.1 von **Figur 3(a)** wurde aus einer grösseren Entfernung aufgenommen und zeigt die ganze Garnschautafel 61, während das Bild 52.2 von **Figur 3(b)** aus einer kleineren Entfernung aufgenommen wurde und Einzelheiten eines Bildgarns 7 bis hin zu aus dem Bildgarn 7 heraus ragenden Faserenden ("Haaren") zeigt. Es handelt sich also beim Bild 52.2 von Figur 3(b) wohl um ein Detail der Garnschautafel 61 von Figur 3(a), nicht aber um ein Detail des Bildes 52.1 von Figur 3(a). Vielmehr wurden die beiden Bilder 52.1, 52.2 derselben Garnschautafel 61 einzeln unter unterschiedlichen Bedingungen aufgenommen. Alternativ könnte man Einzelheiten eines Bildes 52 durch eine Vergrösserung des Bildes 52 zur Darstellung bringen, was jedoch eine sehr hohe Auflösung des Bildes 52 voraussetzen würde.

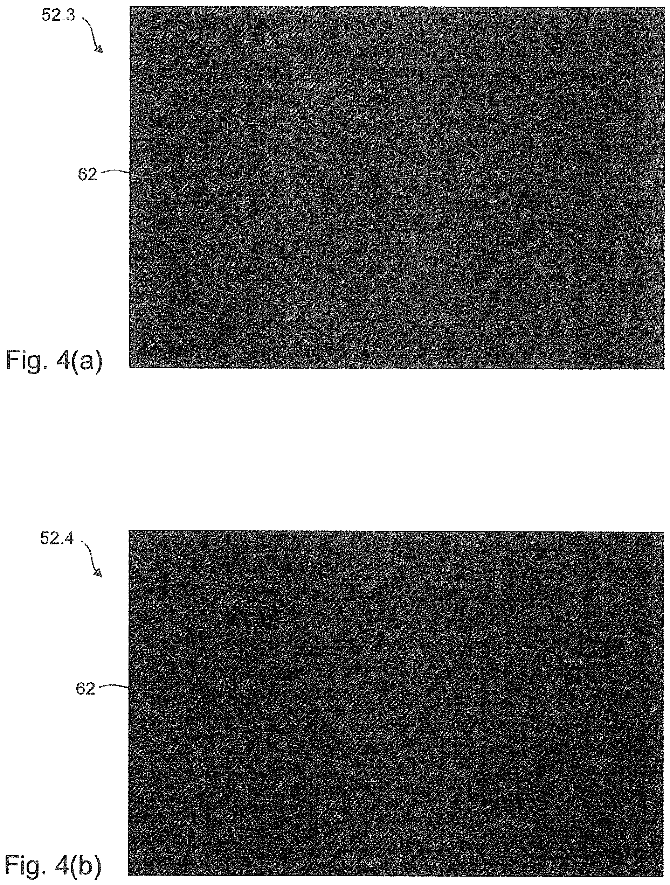

Die Figuren 4 zeigen Bilder 52.3, 52.4 ein und desselben Gewebes 62. Die Bilder 52.3, 52.4 unterscheiden sich voneinander wiederum in der Art ihrer Aufnahme. Das Bild 52.3 von **Figur 4(a)** wurde im Durchlicht, das Bild 52.4 von **Figur 4(b)** im Auflicht aufgenommen. Je nachdem, welche Eigenschaften des Gewebes 62 von Bedeutung sind, eignet sich das eine oder das andere Bild 52.3 bzw. 52.4 besser zur Ausgabe.

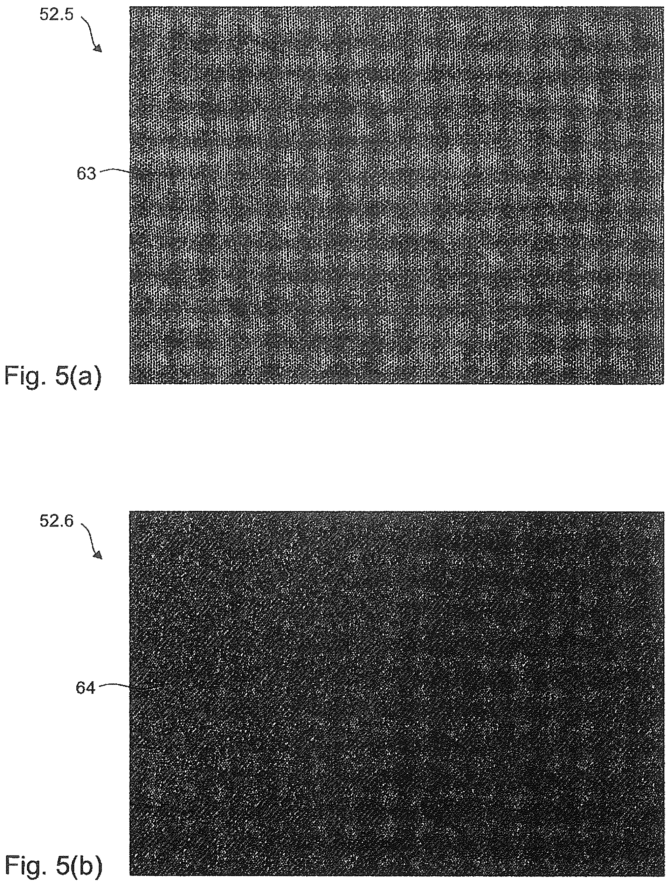

Die Figuren 5 zeigen beispielhaft Bilder 52.5, 52.6 verschiedener Gestricke 63, 64 aus jeweils verschiedenen Bildgarnen 7. Wegen der unterschiedlichen Eigenschaften der für die beiden Gestricke 63, 64 verwendeten Bildgarne 7 hat das Gestrick 63 von **Figur 5(a)** ein schlechteres, "wolkigeres" Aussehen als dasjenige 64 von **Figur 5(b)**.

Die textilen Flächen 61-63 könnten aus demselben Bildgarn 7 hergestellt sein. Dann gehören die Bilder 52.1-52.5 zu einer eine Menge von Bilddatensätzen 51, welche Bilder 52.1-52.5 verschiedener textiler Flächen 61-63, aber Metadatengruppen 53 mit denselben Parameterwerten 54, 55 beinhalten.

**Figur 6** illustriert eine Möglichkeit, wie die Parameterwerte 94, 95 des gegebenen Garns 9 mit den Parameterwerten 54, 55 von Bildgarnen 7 verglichen werden können. Aus Resultaten der Messungen am gegebenen Garn 9 wird jeweils ein Parameterwert 94, 95 mindestens eines Garnparameters bestimmt. Der mindestens eine Garnparameter spannt einen Vektorraum 8 auf. Im einfachen Beispiel von Figur 6 handelt es sich um den zweidimensionalen Vektorraum $R^2$ über der Menge der reellen Zahlen, d. h. es werden zwei Garnparameter betrachtet, die entlang einer horizontalen Achse 81 bzw. einer vertikalen Achse 82 eines kartesischen Koordinatensystems aufgetragen werden. Die in Figur 6 gezeigte Ebene kann somit als eine Darstellung eines zweidimensionalen Vektorraums 8 aufgefasst werden. Selbstverständlich ist die Erfindung nicht auf einen zweidimensionalen Vektorraum beschränkt, sondern kann auf einen n-dimensionalen Vektorraum verallgemeinert werden, wobei n = 1, 2, 3, ... eine natürliche Zahl ist, welche die Anzahl verwendeter Parameter angibt.

Die beiden Parameterwerte 94, 95 des gegebenen Garns 9 definieren einen Garnvektor 91 in dem Vektorraum 8. Ebenso definieren die beiden jeweils in einer Metadatengruppe 53 enthaltenen Parameterwerte 54, 55 der Bildgarne 7 Bildvektoren 71-75, von denen einige in Figur 6 eingezeichnet sind. Die entlang einer bestimmten Achse 81 aufgetragenen Parameter 94, 54 beziehen sich jeweils auf dieselbe Eigenschaft der Garne 9, 7, bspw. auf die Anzahl Nissen.

Auf dem Vektorraum 8 wird eine Metrik definiert, die je zwei Vektoren x, y des Vektorraums einen Abstand d($\underline{x}$, $\underline{y}$) zuordnet. Eine solche Metrik kann z. B. durch die Norm erzeugt werden, die jedem Vektor $\underline{x}$ eine Länge |$\underline{x}$| zuordnet. Dann ist der Abstand d(x, y) durch die Länge $\|\underline{x} - \underline{y}\|$ des jeweiligen Differenzvektors ($\underline{x}$ - $\underline{y}$) gegeben:

$$d\left(\underline{x}, \underline{y}\right) = \left\|\underline{x} - \underline{y}\right\| = \sqrt{\left(x_1 - y_1\right)^2 + \left(x_2 - y_2\right)^2} \ .$$

Bei Kenntnis der Erfindung kann der Fachmann andere Metriken aufstellen, die sich für das erfindungsgemässe Verfahren eignen. Eine Variation der obigen Euklidischen Metrik ergibt sich, indem man die Parameterwerte mit geeigneten Gewichtsfaktoren multipliziert, um die Wichtigkeit des jeweiligen Parameters und/oder die absoluten Grössen der jeweiligen Parameterwerte zu berücksichtigen, z. B.:

$$d\left(\underline{x}, \underline{y}\right) = \sqrt{\left(2\left[x_1 - y_1\right]\right)^2 + \left(3\left[x_2 - y_2\right]\right)^2}\,.$$

In diesem Beispiel ist der zweite Parameter stärker gewichtet als der erste.

Die Parameterwerte für einen jeweiligen Parameter können skaliert werden, so dass sie z. B. in dem Intervall [0, 1] oder [-1, 1] liegen.

Die Definition der Metrik kann von weiteren Daten 96 Eingangsdatengruppe 93 abhängig sein, welche von den Parameterwerten 94, 95 verschieden sind. Solche weiteren Daten 96 können sich insbesondere auf die Erzeugung des Bildes 52 beziehen. Nehmen wir beispielsweise an, die Eingangsdatengruppe 93 enthalte einen ersten Parameterwert 94 für den Garnparameter "Anzahl Nissen" und einen zweiten Parameterwert 95 für den Garnparameter "Haarigkeit". Ein aus grösserer Entfernung aufgenommenes Gesamtbild 52.1 einer textilen Fläche 61, wie es etwa in Figur 3(a) gezeigt ist, wird zwar von den Nissen, aber kaum von der Haarigkeit beeinflusst. Im Gegensatz dazu ist bei einer aus kleinerer Entfernung aufgenommenen Detailansicht 52.2, wie sie etwa in Figur 3(b) gezeigt ist, die Haarigkeit von Interesse, die Nissen hingegen kaum. Deshalb können bei einer Auswahl des Gesamtbildes 52.1 bzw. der Detailansicht 52.2 unterschiedliche Metriken zur Anwendung kommen. Bei der Definition der Metrik wird im ersteren Fall der Garnparameter "Anzahl Nissen" stärker gewichtet, im letzteren Fall der Garnparameter "Haarigkeit". Im Extremfall geht ein unwichtiger Garnparameter überhaupt nicht in die Metrik ein, d. h. er wird mit dem Gewichtsfaktor Null gewichtet.

Erfindungsgemäss werden Abstände d(x, y) zwischen dem Garnvektor 91 einerseits und den Bildvektoren 71-75 andererseits bestimmt und miteinander verglichen. In einer Ausführungsform des erfindungsgemässen Verfahrens wird aus der Bilddatenbank 5 derjenige Bilddatensatz 51 ausgewählt, für welchen der Abstand d($\underline{x}$, $\underline{y}$) am kleinsten ist. Mit anderen Worten ausgedrückt: Es wird derjenige Bilddatensatz 51 ausgewählt, dessen Parameterwerte 54, 55 am nächsten bei den Parameterwerten 94, 95 liegen. Dies trifft im Beispiel von Figur 6 auf den Bilddatensatz 51 mit dem Bildvektor 73 zu. Das entsprechende Bild 52, das in dem ausgewählten Bilddatensatz 51 enthalten ist, wird ausgegeben. Dieses Bild 52 stellt die Vorhersage des Aussehens einer aus dem gegebenen Garn 9 herzustellenden textilen Fläche dar.

In der obigen Diskussion der Ausführungsform von Figur 6 wurden der Anschaulichkeit halber Parameterwerte 94, 95, 54, 55 des gegebenen Garns 9 bzw. des Bildgarns 7 zur Illustration verwendet. Der Vektorraum 8 braucht aber nicht oder nicht ausschliesslich von messbaren Parametern aufgespannt zu werden. Vielmehr können auch weitere Daten 96 aus der Eingangsdatengruppe 93 und weitere Daten 56-58 aus der Metadatengruppe 53 den Vektorraum 8 aufspannen. Falls solche weiteren Daten 96, 56-58 an sich mathematisch nicht handhabbar sind, können ihnen auf eine geeignete Weise reelle Zahlenwerte zugeordnet werden. In einem Beispiel, in dem sich die weiteren Daten auf das Herstellungsverfahren der textilen Fläche beziehen, kann eine solche Zuordnung wie folgt aussehen:

- Garnschautafel → 0.2
- Gewebe → 0.4
- Gestrick → 0.7
- Gewirke → 0.8.

Alternativ können die weiteren Daten 96, 56-58 durch entsprechende Gewichtsfaktoren in der Metrik berücksichtigt werden. In einer weiteren Alternative können die weiteren Daten 96, 56-58 ein Auswahlkriterium für Datensätze 51 bilden. Wenn z. B. die weiteren Daten 96 angeben, dass das gegebene Garn 9 für ein Gestrick verwendet werden soll, so kann es sinnvoll sein, nur diejenigen Datensätze 51 aus der Datenbank 5 zu berücksichtigen, die Bilder 52 von Gestricken zeigen.

Zusätzlich zu dem Bild 52 kann ein aus dem Abstand berechneter Gütewert Q ausgegeben werden, der ein Mass für eine Güte der Vorhersage ist. Der Gütewert Q kann z. B. wie folgt berechnet werden:

$$Q = 1 - d/d_{max}\,,$$

worin $d_{max}$ den maximal möglichen Abstand bezeichnet. Der obige Gütewert Q liegt im Intervall [0, 1], wobei Gütewerte Q nahe bei null schlechte, unzuverlässige Vorhersagen, Gütewerte nahe bei eins gute, zuverlässige Vorhersagen bedeuten. Bei tiefen Gütewerten Q kann zusätzlich zum Bild 52 eine Warnmeldung ausgegeben werden, wie z. B.: "Achtung, eine gute Vorhersage ist nicht möglich."

**Figur 7** zeigt ein Flussdiagramm einer Ausführungsform des erfindungsgemässen Verfahrens zur Erzeugung einer Bilddatenbank 5, wie sie anlässlich von Figur 2 beschrieben wurde. Ein Bildgarn 7 wird bereitgestellt 101. Das Bildgarn 7 wird ausgemessen 102. Aus mindestens einem Resultat der Messung 102 wird jeweils ein Parameterwert 54, 55 mindestens eines Garnparameters des Bildgarns 7 bestimmt 103. Eine textile Fläche 61-64 wird aus dem Bildgarn 7 hergestellt 104. Ein Bild 52 der textilen Fläche 61-64 wird erzeugt 105, z. B. durch ein fotografisches Verfahren aufgenommen. Eine Metadatengruppe 53, die sich zumindest teilweise auf das Bildgarn 7 bezieht, wird gebildet 106. Die Metadatengruppe 53 beinhaltet die Parameterwerte 54, 55 des Bildgarns 7, die einen Bildvektor 71-75 in einem Vektorraum 8 bilden (siehe Figur 6), wobei auf dem Vektorraum 8 eine Metrik definierbar ist, die je zwei Vektoren des Vektorraums 8 einen Abstand zuordnet. Die Metadatengruppe 53 wird dem Bild 52 zugeordnet 107, so dass das Bild 52 und die Metadatengruppe 53 einen Bilddatensatz 51 bilden. Weitere Bilder 52 derselben textilen Fläche 61-64 können erzeugt werden 108, die sich z. B. in der Art ihrer Aufnahme unterscheiden. Eine weitere textile Fläche 61-64 kann aus demselben Bildgarn 7 hergestellt werden 109. Ferner können weitere Bildgarne 7 bereitgestellt 110 und mit diesen die oben beschriebenen Verfahrensschritte 101-110 wiederholt werden. Schliesslich wird die Bilddatenbank 5 aus mindestens 100, vorzugsweise mindestens 1000 und bspw. mindestens 10'000 Bilddatensätzen (51) erzeugt 111.

Nach einer ersten Erzeugung 111 der Bilddatenbank 5 kann die Bilddatenbank 5 geändert werden. Eine Änderung der Bilddatenbank 5 kann z. B. ein Hinzufügen von weiteren Bilddatensätzen 51 oder ein Löschen von nicht mehr gebrauchten Bilddatensätzen 51 beinhalten.

**Figur 8** zeigt ein Flussdiagramm einer ersten Ausführungsform des erfindungsgemässen Verfahrens zur Vorhersage des Aussehens einer aus einem gegebenen Garn 9 herzustellenden textilen Fläche 61-64. Die Bilddatenbank 5, deren Aufbau anlässlich von Figur 2 und deren Erzeugung anlässlich von Figur 7 beschrieben wurden, wird bereitgestellt 201. In der Bilddatenbank 5 beinhaltet jeder Bilddatensatz 51 ein Bild 52 einer aus mindestens einem Bildgarn 7 hergestellten textilen Fläche 61-64 und eine dem Bild 52 zugeordnete Metadatengruppe 53. Jede der Metadatengruppen 53 beinhaltet Parameterwerte 54, 55 des Bildgarns 7, die einen Bildvektor 71-75 in einem Vektorraum 8 (siehe Figur 6) bilden. Eine Messung am gegebenen Garn 9 wird durchgeführt 202. Aus mindestens einem Resultat der Messung 202 wird jeweils ein Parameterwert 94, 95 mindestens eines Garnparameters des gegebenen Garns 9 bestimmt 203. Auf dem Vektorraum 8 wird eine Metrik definiert 204, die je zwei Vektoren des Vektorraums 8 einen Abstand zuordnet. Eine sich zumindest teilweise auf das gegebene Garn 9 beziehende Eingangsdatengruppe 93 wird gebildet 205, welche die Parameterwerte 94, 95 des gegebenen Garns 9 beinhaltet. Diese Parameterwerte 94, 95 bilden einen Garnvektor 91 in demselben Vektorraum 8. Abstände zwischen dem Garnvektor 91 einerseits und den Bildvektoren 71-75 andererseits werden bestimmt 206 und miteinander verglichen 207.

Aufgrund des Vergleichs 207 wird ein Bilddatensatz 51, und vorzugsweise genau ein Bilddatensatz 51, aus der Bilddatenbank 5 ausgewählt 208. Das in dem ausgewählten Bilddatensatz 51 enthaltene Bild 52 wird ausgegeben 209. Es stellt die Vorhersage des Aussehens einer aus dem gegebenen Garn 9 herzustellenden textilen Fläche dar.

Die letzten vier Schritte 206-209 werden vorzugsweise von einem Computer 2, 4 ausgeführt.

**Figur 9** zeigt ein Flussdiagramm einer zweiten Ausführungsform des erfindungsgemässen Verfahrens zur Vorhersage des Aussehens einer textilen Fläche. Darin wird eine bevorzugte Ausführungsform des Vergleichsschrittes 207 und des Auswahlschrittes 208 von Figur 8 diskutiert.Die Schritte der Datenbankbereitstellung 201, der Garnmessung 202, der Parameterwertbestimmung 203 und der Eingangsdatengruppenbildung 205 wurden anlässlich von Figur 8 bereits erklärt. Sie sind in Figur 9 in einem Schritt 301 zusammengefasst.

In der nachfolgend beschriebenen Verfahrensschleife werden die Abstände zwischen dem Garnvektor 91 (siehe Figur 6) einerseits und den Bildvektoren 71-75 andererseits bestimmt und miteinander verglichen.. In der Ausführungsform von Figur 9 und in der folgenden Beschreibung erfolgen die Bestimmung und der Vergleich sequenziell; alternativ könnten sie zumindest teilweise parallel erfolgen. Aus der Bilddatenbank 5 wird ein Bilddatensatz 51 geholt 302. Die Metadatengruppe 53 des Bilddatensatzes 51 enthält z. B. Parameterwerte 54, 55 des betreffenden Bildgarns 7, die einen Bildvektor 71 definieren. Der Abstand zwischen dem Garnvektor 91 und dem aktuellen Bildvektor 71, der dem aus der Bilddatenbank 5 geholten Bilddatensatz 51 entnommen ist, wird berechnet 303. Wenn der aktuell berechnete Abstand der kleinste bisher berechnete Abstand ist 304, wird das aktuelle Bild 52, das dem aus der Bilddatenbank 5 geholten Bilddatensatz 51 entnommen ist, zur Ausgabe bereitgelegt 305. Andernfalls wird die Schleife mit dem nächsten Bilddatensatz 51 durchlaufen, usw. Wenn alle Bilddatensätze 51 geprüft worden sind 306, wird die Schleife beendet. Dasjenige Bild 52, das dann zur Ausgabe bereitliegt, gehört zu demjenigen Bilddatensatz 51, dessen Bildvektor 73 den kleinsten Abstand zum Garnvektor 91 hat. Dieses Bild 52 wird als Resultat des Verfahrens ausgegeben 307. Der Ausgabeschritt 307 von Figur 9 entspricht wiederum dem Ausgabeschritt 209 von Figur 8.

Das Verfahren wird, mit eventueller Ausnahme des ersten Schrittes 301, vorzugsweise von einem Computer 2, 4 ausgeführt.

Selbstverständlich ist die vorliegende Erfindung nicht auf die oben diskutierten Ausführungsformen beschränkt. Bei Kenntnis der Erfindung wird der Fachmann weitere Varianten herleiten können, die auch zum Gegenstand der vorliegenden Erfindung gehören, wie sie in den unabhängigen Patentansprüchen definiert ist.

BEZUGSZEICHENLISTE

| | |
|---|---|
| 1 | Garnprüfgerät |
| 11 | Module des Garnprüfgerätes |
| 2 | lokaler Computer |
| 21 | Ausgabeeinheit |
| 31 | Datenübermittlung vom lokalen Computer zur Wolke |
| 32 | Datenübermittlung von der Wolke zum lokalen Computer |
| 4 | Wolke (Cloud Computing), entfernter Computer |
| 41 | Vergleich |

| | |
|---|---|
| 5 | Bilddatenbank |
| 51 | Bilddatensätze |
| 52 | Bild |
| 53 | Metadatengruppe |
| 54, 55 | Parameterwerte des Bildgarns |
| 56-58 | weitere Daten des Bildgarns |
| | |
| 61 | Garnschautafel als textile Fläche |
| 62 | Gewebe als textile Fläche |
| 63, 64 | Gestricke als textile Flächen |
| | |
| 7 | Bildgarn |
| 71-75 | Bildvektoren |
| | |
| 8 | Vektorraum |
| 81 | horizontale Achse |
| 82 | vertikale Achse |
| | |
| 9 | gegebenes Garn |
| 91 | Garnvektor |
| 93 | Eingangsdatengruppe |
| 94, 95 | Parameterwerte des gegebenen Garns |
| 96 | weitere Daten des gegebenen Garns |
| | |
| 101 | Bereitstellen eines Bildgarns |
| 102 | Messung am Bildgarn |
| 103 | Bestimmung eines Parameterwertes des Bildgarns |
| 104 | Herstellung einer textilen Fläche aus dem Bildgarn |
| 105 | Erzeugung eines Bildes der textilen Fläche |
| 106 | Bildung einer Metadatengruppe |
| 107 | Bildung eines Bilddatensatzes |
| 108 | Frage: Soll ein weiteres Bild aufgenommen werden? |
| 109 | Frage: Soll eine weitere textile Fläche hergestellt werden? |
| 110 | Frage: Soll ein weiteres Bildgarn bereitgestellt werden? |
| 111 | Erzeugung der Bilddatenbank |
| 201 | Bereitstellung einer Bilddatenbank |
| 202 | Messung am gegebenen Garn |
| 203 | Bestimmung einer Eingangsdatengruppe |
| 204 | Definition einer Metrik |
| 205 | Bildung einer Eingangsdatengruppe |
| 206 | Bestimmung der Abstände |
| 207 | Vergleich der Abstände |
| 208 | Auswahl eines Bilddatensatzes aufgrund des Vergleichs |
| 209 | Ausgabe des Bildes |
| | |
| 301 | Ausmessen des gegebenen Garns |
| 302 | Holen des nächsten Bilddatensatzes |
| 303 | Berechnen des Abstandes zwischen dem Garnvektor und dem aktuellen Bildvektor |
| 304 | Frage: Ist dies der kleinste Abstand? |
| 305 | Bereitlegen des aktuellen Bildes zur Ausgabe |
| 306 | Frage: Alle Bilddatensätze geprüft? |

| | |
|---|---|
| 307 | Ausgabe des bereitgelegten Bildes |

**Patentansprüche**

1. Verfahren zur Vorhersage des Aussehens einer aus einem gegebenen Garn (9) herzustellenden textilen Fläche, wobei

   eine Bilddatenbank (5) von Bilddatensätzen (51) bereitgestellt wird (201), in welcher Bilddatenbank (5) jeder Bilddatensatz (51) ein Bild (52) einer aus mindestens einem Bildgarn (7) hergestellten textilen Fläche (61-64) und eine dem Bild (52) zugeordnete Metadatengruppe (53) beinhaltet,

   **dadurch gekennzeichnet, dass**

   jede der Metadatengruppen (53) eine erste Menge von Daten (54, 55) beinhaltet, die einen Bildvektor (71-75) in einem Vektorraum (8) bilden,

   auf dem Vektorraum (8) eine Metrik definiert wird (204), die je zwei Vektoren des Vektorraums (8) einen Abstand zuordnet,

   eine sich zumindest teilweise auf das gegebene Garn (9) beziehende Eingangsdatengruppe (93) gebildet wird (205), die eine zweite Menge von Daten (94, 95) beinhaltet, welche Daten (94, 95) einen Garnvektor (91) in demselben Vektorraum (8) bilden,

   Abstände zwischen dem Garnvektor (91) einerseits und den Bildvektoren (71-75) andererseits bestimmt und miteinander verglichen werden (207),

   aufgrund des Vergleichs (207) ein Bilddatensatz (51) aus der Bilddatenbank (5) ausgewählt wird (208) und das in dem ausgewählten Bilddatensatz (51) enthaltene Bild (52) ausgegeben wird (209).

2. Verfahren nach Anspruch 1, wobei die Eingangsdatengruppe (93) und/oder die Metadatengruppen (53) Daten (94-96, 54-58) beinhalten, die sich auf mindestens einen der folgenden Aspekte beziehen: mindestens ein Parameterwert (94, 95; 54, 55) eines Garnparameters des gegebenen Garns (9) bzw. des Bildgarns (7), für die Herstellung des gegebenen Garns (9) bzw. des Bildgarns (7) verwendetes Rohmaterial, Herstellung des gegebenen Garns (9) bzw. des Bildgarns (7), Herstellung (104) der textilen Fläche (61-64), Erzeugung (105) des Bildes (52).

3. Verfahren nach Anspruch 2, wobei die Eingangsdatengruppe (93) und jede der Metadatengruppen (53) jeweils einen Parameterwert (94, 95; 54, 55) mindestens eines Garnparameters des gegebenen Garns (9) bzw. des Bildgarns (7) beinhalten, welcher mindestens eine Garnparameter sich auf mindestens eine der folgenden Eigenschaften des gegebenen Garns (9) bzw. des Bildgarns (7) bezieht: Masse, Querdimension, Anzahl Nissen, Garnnummer, Haarigkeit, Fremdstoffgehalt, Reissfestigkeit, Dehnung, Drehung, Reibung.

4. Verfahren nach Anspruch 3, wobei eine Messung am gegebenen Garn (9) durchgeführt wird (202) und der mindestens eine Parameterwert (94, 95) des gegebenen Garns (9) aus mindestens einem Resultat der Messung (202) und vorzugsweise durch eine anschliessende statistische Auswertung von Resultaten der Messung (202) bestimmt wird.

5. Verfahren nach einem der vorangehenden Ansprüche, wobei die Eingangsdatengruppe (93) Daten (96) beinhaltet, die sich auf mindestens einen der folgenden Aspekte beziehen: für die Herstellung des gegebenen Garns (9) verwendetes Rohmaterial, Herstellung des gegebenen Garns (9), vorgesehene Herstellung der textilen Fläche, Erzeugung (105) des Bildes (52), und wobei die Definition (204) der Metrik von den genannten Daten (96) abhängig ist.

6. Verfahren nach einem der vorangehenden Ansprüche, wobei derjenige Bilddatensatz (51) ausgewählt wird (208), für dessen Bildvektor (73) der Abstand am kleinsten ist.

7. Verfahren nach einem der vorangehenden Ansprüche , wobei zusätzlich zu dem Bild (52) ein aus dem Abstand berechneter Gütewert ausgegeben wird, der ein Mass für eine Güte der Vorhersage ist.

8. Verfahren nach einem der vorangehenden Ansprüche, wobei die Verfahrensschritte der Bestimmung (206) und des Vergleichs (207) der Abstände zwischen dem Garnvektor (91) und den Bildvektoren (71-75),
der Auswahl (208) eines Bilddatensatzes (51) aus der Bilddatenbank (5) aufgrund des Vergleichs (207) und
der Ausgabe (209) des in dem ausgewählten Bilddatensatz (51) enthaltenen Bildes (52)
von einem Computer (2, 4) ausgeführt werden.

9. Verfahren nach Anspruch 8, wobei Cloud Computing angewendet wird, indem die Eingangsdatengruppe (93) von einem lokalen Computer (2) über ein Netzwerk an mindestens einen entfernten Computer (4) übermittelt wird (31), der Vergleich (207) und die Auswahl (208) durch den mindestens einen entfernten Computer (4) erfolgt, das ausgewählte Bild (52) über das Netzwerk von dem mindestens einen entfernten Computer (4) an den lokalen Computer (2) übermittelt wird (32) und das ausgewählte Bild (52) von dem lokalen Computer (2) ausgegeben wird (209).

10. Computerprogrammprodukt mit auf einem maschinenlesbaren Träger gespeichertem Programmcode zur Durchführung der in Anspruch 8 genannten Verfahrensschritte (206-209), wenn das Computerprogrammprodukt auf einem Computer (2, 4) abläuft.

11. Bilddatenbank (5) von Bilddatensätzen (51) zur Verwendung im Verfahren nach einem der Ansprüche 1-9, wobei
jeder Bilddatensatz (51) ein Bild (52) einer aus mindestens einem Bildgarn (7) hergestellten textilen Fläche (61-64) und eine dem Bild (52) zugeordnete Metadatengruppe (53) beinhaltet,
jede der Metadatengruppen (53) eine erste Menge von Daten (54, 55) beinhaltet, die einen Bildvektor (71-75) in einem Vektorraum (8) bilden, wobei auf dem Vektorraum (8) eine Metrik definierbar ist, die je zwei Vektoren des Vektorraums (8) einen Abstand zuordnet, und
die Bilddatenbank (5) mindestens 100, vorzugsweise mindestens 1000 und bspw. mindestens 10'000 Bilddatensätze (51) enthält.

12. Bilddatenbank (5) nach Anspruch 11, wobei die Metadatengruppen (53) Daten (54-58) beinhalten, die sich auf mindestens einen der folgenden Aspekte beziehen: mindestens ein Parameterwert (54, 55) eines Garnparameters des Bildgarns (7), für die Herstellung des Bildgarns (7) verwendetes Rohmaterial, Herstellung des Bildgarns (7), Herstellung (104) der textilen Fläche (61-64), Erzeugung (105) des Bildes (52).

13. Bilddatenbank (5) nach Anspruch 12, wobei jede der Metadatengruppen (53) jeweils einen Parameterwert (54, 55) mindestens eines Garnparameters des Bildgarns (7) beinhaltet, welcher mindestens eine Garnparameter sich auf mindestens eine der folgenden Eigenschaften des Bildgarns (7) bezieht: Masse, Querdimension, Anzahl Nissen, Garnnummer, Haarigkeit, Fremdstoffgehalt, Reissfestigkeit, Dehnung, Drehung, Reibung.

14. Verfahren zur Erzeugung einer Bilddatenbank (5) von Bilddatensätzen (51) zur Verwendung im Verfahren nach einem der Ansprüche 1-9, wobei
Bilder (52) mindestens einer aus mindestens einem Bildgarn (7) hergestellten textilen Fläche (61-64) erzeugt werden (105),
Bilddatensätze (51) gebildet werden (107), wobei jeder Bilddatensatz (51) eines der Bilder (52) und eine dem Bild (52) zugeordnete Metadatengruppe (53) beinhaltet, jede der Metadatengruppen (53) eine erste Menge von Daten (54, 55) beinhaltet, die einen Bildvektor (71-75) in einem Vektorraum (8) bilden, wobei auf dem Vektorraum (8) eine Metrik definierbar ist, die je zwei Vektoren des Vektorraums (8) einen Abstand zuordnet, und
die Bilddatenbank (5) aus mindestens 100, vorzugsweise mindestens 1000 und bspw. mindestens 10'000 Bilddatensätzen (51) erzeugt wird (111).

**15.** Verfahren nach Anspruch 14, wobei die Metadatengruppen (53) Daten (54-58) beinhalten, die sich auf mindestens einen der folgenden Aspekte beziehen: mindestens ein Parameterwert eines Garnparameters des Bildgarns (7), für die Herstellung des Bildgarns (7) verwendetes Rohmaterial, Herstellung des Bildgarns (7), Herstellung (104) der textilen Fläche (61-64), Erzeugung (105) der Bilder (52).

**16.** Verfahren nach Anspruch 15, wobei jede der Metadatengruppen (53) jeweils einen Parameterwert (54, 55) mindestens eines Garnparameters des Bildgarns (7) beinhaltet, welcher mindestens eine Garnparameter sich auf mindestens eine der folgenden Eigenschaften des Bildgarns (7) bezieht: Masse, Querdimension, Anzahl Nissen, Garnnummer, Haarigkeit, Fremdstoffgehalt, Reissfestigkeit, Dehnung, Drehung, Reibung.

**17.** Verfahren nach Anspruch 16, wobei eine Messung am Bildgarn (7) durchgeführt wird (102) und der mindestens eine Parameterwert (54, 55) des Bildgarns (7) aus mindestens einem Resultat der Messung (102) und vorzugsweise durch eine anschliessende statistische Auswertung von Resultaten der Messung (102) bestimmt wird.

**18.** Verfahren nach einem der Ansprüche 14-17, wobei mehrere verschiedene textile Flächen (61-64) aus demselben Bildgarn (7) hergestellt werden (104), so dass die Bilddatenbank (5) eine Menge von Bilddatensätzen (51) enthält, die Bilder (52) verschiedener textiler Flächen (61-64), aber Metadatengruppen (53) mit gleichen ersten Mengen von Daten (54, 55) beinhalten.

**19.** Verfahren nach Anspruch 18, wobei sich die textilen Flächen (61-64) mit gleichen ersten Mengen von Daten (54, 55) durch mindestens einen der folgenden Unterscheidungsaspekte voneinander unterscheiden: Herstellungsverfahren der textilen Fläche (61-64), Typ einer die textile Fläche (61-64) herstellenden Maschine, und wobei der mindestens eine Unterscheidungsaspekt in den entsprechenden Metadatengruppen (53) abgebildet ist.

**20.** Verfahren nach einem der Ansprüche 14-19, wobei mehrere verschiedene Bilder (52.1, 52.2) derselben textilen Fläche (61) erzeugt werden (105).

**21.** Verfahren nach einem der Ansprüche 14-20, wobei die Bilder (52) durch ein fotografisches Verfahren aufgenommen werden (105).

**22.** Verfahren nach den Ansprüchen 20 und 21, wobei die verschiedenen Bilder (52.1, 52.2) jeweils durch ein fotografisches Verfahren aufgenommen werden (105),

die verschiedenen Bilder (52.1, 52.2) derselben textilen Fläche (61) sich durch mindestens einen der folgenden Unterscheidungsaspekte voneinander unterscheiden:

Art der Beleuchtung der textilen Fläche bei der Bildaufnahme (105), gegenseitige Lage einer Kamera und der textilen Fläche (61) bei der Bildaufnahme (105), bei der Bildaufnahme (105) eingestellte fototechnische Parameter, und der mindestens eine Unterscheidungsaspekt in den entsprechenden Metadatengruppen (53) abgebildet ist.

**Claims**

**1.** A method for forecasting the appearance of a textile surface to be produced from a given yarn (9), wherein an image database (5) of image data records (51) is provided (201), in which image database (5) each image data record (51) includes an image (52) of a textile surface (61-64) produced from at least one image yarn (7) and a metadata group (53) associated with the image (52),
**characterized in that**
each of the metadata groups (53) includes a first set of data (54, 55) forming an image vector (71-75) in a vector space (8),
on the vector space (8), a metric is defined (204) which assigns a distance to two vectors each of the vector space (8),
an input data group (93), at least partially related to the given yarn (9), is formed (209), which includes a second set of data (94, 95), which data (94, 95) forms a yarn vector (91) in the same vector space (8),
distances between the yarn vector (91) on the one hand and the image vectors (71-75) on the other hand are determined and compared (207) with each other,
based on the comparison (207), an image data record (51) is selected (208) from the image database (5) and
the image (52) contained in the selected image data record (51) is output (209).

**2.** A method according to claim 1, wherein the input data group (93) and/or the metadata groups (53) contain data (94-96, 54-58) relating to at least one of the following aspects: at least one parameter value (94, 95; 54, 55) of a yarn parameter of the given yarn (9) or image yarn (7), raw material used for the production of the given yarn (9) or image yarn (7), production of the given yarn (9) or image yarn (7), production (104) of the textile surface (61-64), generation (105) of the image (52).

**3.** A method according to claim 2, wherein the input data group (93) and each of the metadata groups (53) each include a parameter value (94, 95; 54, 55) of at least one yarn parameter of the given yarn (9) and image yarn (7), which at least one yarn parameter relates to at least one of the following properties of the given yarn (9) and image yarn (7): mass, transverse dimension, number of neps, yarn count, hairiness, foreign-matter content, tensile strength, elongation, twist, friction.

4. A method according to claim 3, wherein a measurement is performed (202) on the given yarn (9) and the at least one parameter value (94, 95) of the given yarn (9) is determined from at least one result of the measurement (202) and preferably by a subsequent statistical evaluation of results of the measurement (202).

5. A method according to one of the preceding claims, wherein the input data group (93) includes data (96) relating to at least one of the following aspects: raw material used for the production of the given yarn (9), production of the given yarn (9), intended production of the textile surface, generation (105) of the image (52), and wherein the definition (204) of the metric depends on said data (96).

6. A method according to one of the preceding claims, wherein the image data record (51) is selected (208) for the image vector (73) of which the distance is the smallest.

7. A method according to one of the preceding claims, wherein in addition to the image (52) a quality value calculated from the distance is output, which is a measure of a quality of the forecast.

8. A method according to one of the preceding claims, wherein the method steps of the determination (206) and the comparison (207) of the distances between the yarn vector (91) and the image vectors (71-75), the selection (208) of an image data record (51) from the image database (5) on the basis of the comparison (207) and
the output (209) of the image (52) contained in the selected image data record (51)
are carried out by a computer (2, 4).

9. A method according to claim 8, wherein cloud computing is applied by transmitting the input data group (93) from a local computer (2) to at least one remote computer (4) via a network (31), the comparison (207) and the selection (208) are carried out by the at least one remote computer (4), the selected image (52) is transmitted (32) via the network from the at least one remote computer (4) to the local computer (2) and the selected image (52) is output (209) by the local computer (2).

10. A computer program product having program code stored on a machine-readable carrier for carrying out the method steps (206-209) mentioned in claim 8 when the computer program product runs on a computer (2, 4).

11. An image database (5) of image data records (51) for use in the method according to one of the claims 1-9, wherein
each image data record (51) contains an image (52) of a textile surface (61-64) produced from at least one image yarn (7) and a metadata group (53) associated with the image (52),
each of the metadata groups (53) includes a first set of data (54, 55) forming an image vector (71-75) in a vector space (8), wherein a metric is definable on the vector space (8), which metric assigns a distance to two vectors each of the vector space (8), and
the image database (5) contains at least 100, preferably at least 1,000 and, for example, at least 10,000 image data records (51).

12. An image database (5) according to claim 11, wherein the metadata groups (53) include data (54-58) relating to at least one of the following aspects: at least one parameter value (54, 55) of a yarn parameter of the image yarn (7), raw material used for the production of the image yarn (7), production of the image yarn (7), production (104) of the textile surface (61-64), generation (105) of the image (52).

13. An image database (5) according to claim 12, wherein each of the metadata groups (53) each includes a parameter value (54, 55) of at least one yarn parameter of the image yarn (7), which at least one yarn parameter relates to at least one of the following properties of the image yarn (7): mass, transverse dimension, number of neps, yarn count, hairiness, foreign-matter content, tensile strength, elongation, twist, friction.

14. A method of generating an image database (5) of image data records (51) for use in the method according to one of the claims 1-9, wherein
images (52) of at least one textile surface (61-64) produced from at least one image yarn (7) are produced (105),
image data records (51) are formed (107), wherein each image data record (51) contains one of the images (52) and one metadata group (53) associated with the image (52),
each of the metadata groups (53) includes a first set of data (54, 55) forming an image vector (71-75) in a vector space (8), wherein a metric is definable on the vector space (8), which assigns a distance to two vectors each of the vector space (8), and
the image database (5) is generated (111) from at least 100, preferably at least 1,000 and, for example, at least 10,000 image data records (51).

15. A method according to claim 14, wherein the metadata groups (53) include data (54-58) relating to at least one of the following aspects: at least one parameter value of a yarn parameter of the image yarn (7), raw material used to produce the image yarn (7), production of the image yarn (7), production (104) of the textile surface (61-64), generation (105) of the images (52).

16. A method according to claim 15, wherein each of the metadata groups (53) in each case includes a parameter value (54, 55) of at least one yarn parameter of the image yarn (7), which at least one yarn parameter relates to at least one of the following properties of the image yarn (7): transverse dimension, number of neps, yarn count, hairiness, foreign-matter content, tensile strength, elongation, twist, friction.

17. A method according to claim 16, wherein a measurement (102) is performed on the image yarn (7) and the at least one parameter value (54, 55) of the image yarn (7) is determined from at least one result of the measurement (102) and preferably by a subsequent statistical evaluation of results of the measurement (102).

18. A method according to one of the claims 14-17, wherein a plurality of different textile surfaces (61-64) are produced (104) from the same image yarn (7), so that the image database (5) contains a set of image data records (51) containing images (52) of different textile surfaces (61-64), but including metadata groups (53) with the same first sets of data (54, 55).

19. A method according to claim 18, wherein the textile surfaces (61-64) having the same first sets of data (54, 55) differ from each other by at least one of the following differentiation aspects: manufacturing method of the textile surface (61-64), type of a machine producing the textile surface (61-64), and wherein the at least one differentiation aspect is mapped in the respective metadata groups (53).

20. A method according to one of the claims 14-19, wherein a plurality of different images (52.1, 52.2) of the same textile surface (61) is created (105).

21. A method according to one of the claims 14-20, wherein the images (52) are taken by a photographic process (105).

22. A method according to claims 20 and 21, wherein the different images (52.1, 52.2) are each taken by a photographic process (105),

the different images (52.1, 52.2) of the same textile surface (61) differ from one another by at least one of the following differentiation aspects: type of illumination of the textile surface during image recording (105), mutual position of a camera and the textile surface (61) during image recording (105), phototechnical parameters set during image recording (105), and

the at least one differentiation aspect is mapped in the corresponding metadata groups (53).

## Revendications

1. Procédé pour prédire l'apparence d'une surface textile à produire à partir d'un fil donné (9), dans lequel une banque de données d'images (5) composée d'ensembles de données d'images (51) est préparée (201), chaque ensemble de données d'images (51) de cette banque de données d'images (5) contenant une image (52) d'une surface textile (61-64) produite à partir d'au moins un fil d'image (7) et un groupe de métadonnées (53) associé à l'image (52),
**caractérisé en ce que**
chacun des groupes de métadonnées (53) contient un premier volume de données (54, 55) qui forment un vecteur d'image (71-75) dans un espace vectoriel (8),
un paramètre de mesure associant une distance aux vecteurs de l'espace vectoriel (8) deux par deux est défini (204) dans l'espace vectoriel (8),
un groupe de données d'entrée (93) se rapportant au moins en partie au fil donné (9) et contenant un deuxième volume de données (94, 95) est formé (205), lesquelles données (94, 95) forment un vecteur de fil (91) dans le même espace vectoriel (8),
les distances entre le vecteur de fil (91) et les vecteurs d'image (71-75) sont déterminées et comparées entre elles (207),
un ensemble de données d'image (51) est choisi (208) sur la base de la comparaison (207) dans la banque de données d'images (5) et
l'image (52) contenue dans l'ensemble de données d'image (51) choisi est émise en sortie (209).

2. Procédé selon la revendication 1, dans lequel le groupe de données d'entrée (93) et/ou les groupes de métadonnées (53) contiennent des données (94-96, 54-58) qui se rapportent à au moins un des aspects suivants : au moins une valeur de paramètre (94, 95 ; 54, 55) d'un paramètre de fil du fil donné (9) ou du fil d'image (7), la matière première utilisée pour fabriquer le fil donné (9) ou le fil d'image (7), la fabrication du fil donné (9) ou du fil d'image (7), la fabrication (104) de la surface textile (61-64), la génération (105) de l'image (52).

3. Procédé selon la revendication 2, dans lequel le groupe de données d'entrée (93) et chacun des groupes de métadonnées (53) contiennent chacun une valeur de paramètre (94, 95 ; 54, 55) d'au moins un paramètre de fil du fil donné (9) ou du fil d'image (7), lequel au moins un paramètre de fil se rapporte à au moins une des propriétés suivantes du fil donné (9) ou du fil d'image (7) : masse, dimension transversale, nombre de neps, numéro de fil, ébouriffage, teneur en matières étrangères, résistance à la déchirure, allongement, vrillage, friction.

4. Procédé selon la revendication 3, dans lequel une mesure est effectuée (202) sur le fil donné (9) et l'au moins une valeur de paramètre (94, 95) du fil donné (9) est déterminée à partir d'au moins un résultat de la mesure (202) et de préférence par une analyse statistique subséquente des résultats de la mesure (202).

5. Procédé selon l'une des revendications précédentes, dans lequel le groupe de données d'entrée (93) contient des données (96) qui se rapportent à au moins un des aspects suivants : matière première utilisée pour la fabrication du fil donné (9), fabrication du fil donné (9), fabrication prévue de la surface textile, génération (105) de l'image (52), et dans lequel la définition (204) du paramètre de mesure dépend desdites données (96).

6. Procédé selon l'une des revendications précédentes, dans lequel est sélectionné (208) l'ensemble de données d'image (51) dont le vecteur d'image (73) présente la plus petite distance.

7. Procédé selon l'une des revendications précédentes, dans lequel est émise en sortie, outre l'image (52), une valeur de qualité calculée à partir de la distance, qui constitue une mesure pour la qualité de la prédiction.

8. Procédé selon l'une des revendications précédentes, dans lequel les étapes de procédé de

déterminateur (206) et de comparaison (207) des distances entre le vecteur de fil (91) et les vecteurs d'image (71-75),
sélection (208) d'un ensemble de données d'image (51) à partir de la banque de données d'images (5) sur la base de la comparaison (207) et
sortie (209) de l'image (52) contenue dans l'ensemble de données d'image (51) sélectionné

sont exécutées par un ordinateur (2, 4).

9. Procédé selon la revendication 8, dans lequel un calcul en nuage est utilisé en cela que le groupe de données d'entrée (93) est transmis (31) par un ordinateur local (2), via un réseau, à au moins un ordinateur distant (4), la comparaison (207) et la sélection (208) sont effectuées par l'au moins un ordinateur distant (4), l'image (52) sélectionnée est transmise (32) via le réseau de l'au moins un ordinateur distant (4) à l'ordinateur local (2) et l'image (52) sélectionnée est émise en sortie (209) par l'ordinateur local (2).

10. Programme d'ordinateur avec un code de programme enregistré sur un support lisible par une machine pour la mise en oeuvre des étapes de procédé (206-209) énumérées dans la revendication 8 quand le programme d'ordinateur est exécuté sur un ordinateur (2, 4).

11. Banque de données d'images (5) d'ensembles de données d'images (51) à utiliser dans le procédé selon l'une des revendications 1 à 9, dans lequel chaque ensemble de données d'image (51) contient une image (52) d'une surface textile (61-64) fabriquée à partir d'au moins un fil d'image (7) et un groupe de métadonnées (53) associé à l'image (52), chacun des groupes de métadonnées (53) contient un premier volume de données (54, 55) qui forment un vecteur d'image (71-75) dans un espace vectoriel (8), un paramètre de mesure pouvant être défini dans l'espace vectoriel (8) pour attribuer une distance aux vecteurs de l'espace vectoriel (8) deux par deux, et la banque de données d'images (5) contient au moins 100, de préférence au moins 1000 et, par exemple, au moins 10 000 ensembles de données d'image (51).

12. Banque de données d'images (5) selon la revendication 11, dans laquelle les groupes de métadonnées (53) contiennent des données (54-58) qui se rapportent à au moins un des aspects suivants : au moins une valeur de paramètre (54, 55) d'un paramètre de fil du fil d'image (7), la matière première utilisée pour fabriquer le fil d'image (7), la fabrication du fil d'image (7), la fabrication (104) de la surface textile (61-64), la génération (105) de l'image (52).

13. Banque de données d'images (5) selon la revendication 12, dans laquelle chacun des groupes de métadonnées (53) contient une valeur de paramètre (54, 55) d'au moins un paramètre de fil du fil d'image (7) lequel au moins un paramètre de fil se rapporte à au moins une des propriétés suivantes du fil d'image (7) : masse, dimension transversale, nombre de neps, numéro de fil, ébouriffage, teneur en matières étrangères, résistance à la déchirure, allongement, vrillage, friction.

14. Procédé pour la génération d'une banque de données d'images (5) d'ensembles de données d'images (51) à utiliser dans le procédé selon l'une des revendications 1 à 9, dans lequel

des images (52) d'au moins une surface textile (61-64) produite à partir d'au moins un fil d'image (7) sont générées (105),

des ensembles de données d'image (51) sont constitués (107), chaque ensemble de données d'image (51) contenant l'une des images (52) et un groupe de métadonnées (53) associé à l'image (52),

chacun des groupes de métadonnées (53) contient un premier volume de données (54, 55) qui forment un vecteur d'image (71-75) dans un espace vectoriel (8), un paramètre de mesure associant une distance aux vecteurs de l'espace vectoriel (8) deux par deux étant défini (204) dans l'espace vectoriel (8), et

la banque de données d'images (5) est générée (111) à partir d'au moins 100, de préférence au moins 1000 et, par exemple, au moins 10 000 ensembles de données d'image (51).

15. Procédé selon la revendication 14, dans lequel les groupes de métadonnées (53) contiennent des données (54-58) qui se rapportent à au moins un des aspects suivants : au moins une valeur de paramètre d'un paramètre de fil du fil d'image (7), la matière première utilisée pour fabriquer le fil d'image (7), la fabrication du fil d'image (7), la fabrication (104) de la surface textile (61-64), la génération (105) des images (52).

16. Procédé selon la revendication 15, dans lequel chacun des groupes de métadonnées (53) contient une valeur de paramètre (54, 55) d'au moins un paramètre de fil ou du fil d'image (7), lequel au moins un paramètre de fil se rapporte à au moins une des propriétés suivantes du fil d'image (7) : masse, dimension transversale, nombre de neps, numéro de fil, ébouriffage, teneur en matières étrangères, résistance à la déchirure, allongement, vrillage, friction.

17. Procédé selon la revendication 16, dans lequel une mesure est effectuée (102) sur le fil d'image (7) et l'au moins une valeur de paramètre (54, 55) du fil d'image (7) est déterminée à partir d'au moins un résultat de la mesure (102), de préférence par une analyse statistique subséquente des résultats de la mesure (102).

18. Procédé selon l'une des revendications 14 à 17, dans lequel plusieurs surfaces textiles (61-64) différentes sont fabriquées (104) à partir du même fil d'image (7), de sorte que la banque de données d'images (5) contient un volume d'ensembles de données d'images (51) qui contiennent des images (52) de surfaces textiles (61-64) différentes mais des volumes de métadonnées (53) ayant les mêmes premiers volumes de données (54, 55).

19. Procédé selon la revendication 18, dans lequel les surfaces textiles (61-64) ayant les mêmes premiers volumes de données (54, 55) se différencient par au moins un des aspects de différenciation suivants : méthode de fabrication de la surface textile (61-64), type de machine fabricant la surface textile (61-64), et l'au moins un aspect de différenciation est représenté dans les groupes de métadonnées (53) correspondants.

20. Procédé selon l'une des revendications 14 à 19, dans lequel plusieurs images (52.1, 52.2) différentes de la même surface textile (61) sont créées (105).

21. Procédé selon l'une des revendications 14 à 20, dans lequel les images (52) sont acquises (105) par un procédé photographique.

22. Procédé selon les revendications 20 et 21, dans lequel les différentes images (52.1, 52.2) sont acquises (105) chacune par un procédé photographique, les différentes images (52.1, 52.2) de la même surface textile (61) diffèrent les unes des autres par au moins un des aspects de différenciation suivants : mode d'éclairage de la surface textile lors de la prise de vue (105), position de l'appareil photographique par rapport à la surface textile (61) lors de la prise de vue (105), paramètres photographiques réglés lors de la prise de vue (105), et l'au moins un aspect de différenciation est représenté dans les groupes de métadonnées (53) correspondants.

Fig. 1

Fig. 2

Fig. 6

52.1

61

Fig. 3(a)

52.2

7

Fig. 3(b)

52.3

62

Fig. 4(a)

52.4

62

Fig. 4(b)

Fig. 5(a)

Fig. 5(b)

START

BEREITSTELLUNG EINES BILDGARNS ～ 101

MESSUNG AM BILDGARN ～ 102

BESTIMMUNG EINES PARAMETERWERTES ～ 103

HERSTELLUNG EINER TEXTILEN FLÄCHE
AUS DEM BILDGARN ～ 104

ERZUGUNG EINES BILDES
DER TEXTILEN FLÄCHE ～ 105

BILDUNG EINER METADATENGRUPPE ～ 106

ZUORDNUNG EINER METADATENGRUPPE
ZUM BILD → BILDDATENSATZ ～ 107

108
WEITERES BILD?
JA
NEIN

109
WEITERE TEXTILE FLÄCHE?
JA
NEIN

110
WEITERES BILDGARN?
JA
NEIN

ERZEUGUNG DER BILDDATENBANK ～ 111

ENDE

Fig. 7

START

BEREITSTELLUNG EINER BILDDATENBANK — 201

MESSUNG AM GEGEBENEN GARN — 202

BESTIMMUNG VON PARAMETERWERTEN — 203

DEFINITION EINER METRIK — 204

BILDUNG EINER EINGANGSDATENGRUPPE — 205

BESTIMMUNG DER ABSTÄNDE — 206

VERGLEICH DER ABSTÄNDE — 207

AUSWAHL EINES BILDDATENSATZES
AUFGRUND DES VERGLEICHS — 208

AUSGABE DES BILDES — 209

ENDE

Fig. 8

START

DATENBANK, MESSUNG, PARAMETERWERT, EINGANGSDATENGRUPPE — 301

HOLEN DES NÄCHSTEN BILDDATENSATZES MIT BILDVEKTOR — 302

BERECHNUNG DES ABSTANDES ZWISCHEN GARNVEKTOR UND AKTUELLEM BILDVEKTOR — 303

IST DIES DER KLEINSTE ABSTAND? 304 NEIN

JA

BEREITLEGEN DES AKTUELLEN BILDES ZUR AUSGABE — 305

ALLE BILDDATENSÄTZE GEPRÜFT? 306 NEIN

JA

AUSGABE DES BEREITGELEGTEN BILDES — 307

ENDE

Fig. 9

**IN DER BESCHREIBUNG AUFGEFÜHRTE DOKUMENTE**

**In der Beschreibung aufgeführte Patentdokumente**

- US 5671061 A
- WO 9731262 A1
- WO 9816823 A1
- US 6928335 B1
- EP 1006225 A2
- US 6510734 B1
- WO 2009039668 A1
- US 20080209998 A1
- WO 2012051730 A1

**In der Beschreibung aufgeführte Nicht-Patentliteratur**

- Höhere Mathematik für Ingenieure. Lineare Algebra. Springer Fachmedien, 1990, vol. II